Europäisches Patentamt

**European Patent Office**

**Office européen des brevets**

(11) Veröffentlichungsnummer: **0 058 387**
**A1**

(12) # EUROPÄISCHE PATENTANMELDUNG

(21) Anmeldenummer: **82100990.9**

(51) Int. Cl.³: **C 07 H 15/24**
**A 61 K 31/70**

(22) Anmeldetag: **11.02.82**

(30) Priorität: **14.02.81 DE 3105483**

(43) Veröffentlichungstag der Anmeldung:
**25.08.82 Patentblatt 82/34**

(84) Benannte Vertragsstaaten:
**AT BE CH DE FR GB IT LI LU NL SE**

(71) Anmelder: **BEHRINGWERKE Aktiengesellschaft**
**Postfach 1140**
**D-3550 Marburg 1(DE)**

(72) Erfinder: **Kurrle, Roland, Dr.**
**Schenkendorfweg 18**
**D-3550 Marburg/Lahn(DE)**

(72) Erfinder: **Sedlacek, Hans-Harald, Dr.**
**Am Sonnenhang 3**
**D-3550 Marburg/Lahn(DE)**

(72) Erfinder: **Seiler, Friedrich Robert, Dr.**
**Oberer Eichweg 10**
**D-3550 Marburg/Lahn(DE)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Verwendung von Aclacinomycin als Immunsuppressivum.

(57) Es wird die Verwendung des Zytostatikums und Antibiotikums Aclacinomycin als Immunsuppressivum beschrieben.

EP 0 058 387 A1

BEHRINGWERKE AKTIENGESELLSCHAFT    Hoe 81/B 003
                                   Dr.Ha/Bl.


Verwendung von Aclacinomycin als Immunsuppressivum

Die Erfindung betrifft die Verwendung des Zytostatikums und Antibiotikums Aclacinomycins als Immunsuppressivum.

Die Unterdrückung der immunologischen Reaktion beispielsweise bei hyperergischen Reaktionen (Allergien), bei Immunkomplexerkrankungen, bei Autoimmunerkrankungen und bei Organverpflanzungen stellt heutzutage immer noch ein nicht zufriedenstellend gelöstes Problem dar. Die bislang hierbei verwendeten Zytostatika haben in dem Dosierbereich, in welchem sie immunsuppressiv wirken, entweder starke akute Nebenwirkungen, welche ihre Anwendung einschränken, und/oder sind mutagen und damit mit hoher Wahrscheinlichkeit auch kanzerogen.

Es gibt unter den zahlreichen Zytostatika, welche für die Tumortherapie verwandt werden, vereinzelt auch solche, welche sich durch eine relativ geringe Toxizität und eine fehlende Mutagenität auszeichnen. Zu diesen Zytostatika gehört das zytostatische Antibiotikum Aclacinomycin, welches ein Anthracyclinglykosid darstellt und ist und entsprechend der deutschen Patentschrift 25 32 568 aus Kulturbrühen von Streptocymes galilaeus ATCC 31133 gewonnen wird. Von diesem Zytostatikum war bislang bekannt, daß es zwar tumorstatisch, jedoch nicht, daß es immunsuppressiv wirkt.

Es wurde nun überraschend gefunden, daß bei Verwendung von Dosierungen des Aclacinomycins, die unterhalb derjenigen liegen, die als tumorstatisch anzusehen sind, eine deutliche immunsuppressive Wirkung nachzuweisen ist.

- 2 -

Gegenstand der Erfindung ist somit die Verwendung von Aclacinomycin als Immunsuppressivum.

Diese immunsuppressive Wirkung wird in Abhängigkeit von der untersuchten Tierspezies in einem Dosisbereich von 5 mg bis 0,0001 ug/kg Körpergewicht gefunden.

Ein besonderer Vorteil der Erfindung ist darin zu sehen, daß die immunsuppressive Wirkung gerade bei Anwendung geringer Dosen, nicht jedoch höherer Dosen auftritt, so daß die trotz relativer Verträglichkeit von Aclacinomycin nicht auszuschließenden Nebenwirkungen auf ein Minimum reduziert werden.

Für die Anwendung kann das Aclacinomycin auf an sich bekannte Weise gewünschtenfalls zusammen mit weiteren pharmazeutisch unbedenklichen Zusätzen formuliert und in für orale oder parenterale Verabreichung geeignete Formen gebracht werden. Vorzugsweise befindet es sich in flüssiger, insbesondere wässriger, Form.

Es können löslichkeitsfördernde Zusätze und Lösungsmittel zugesetzt werden, um eine zufriedenstellende Konzentration des Aclacinomycins zu erreichen, beispielsweise Aethanol.

Als weiterer Lösungsvermittler oder weiteres Lösungsmittel kann Benzoesäurebenzylester verwendet werden.

Ein pflanzliches Öl, wie Olivenöl oder Maisöl, kann als pharmazeutischer Träger anwesend sein.

In Emulsion kann ein Lecithin wie Sojalecithin anwesend sein.

- 3 -

Die Menge des in einer pharmazeutischen Zubereitung zu verabreichenden Aclacinomycins hängt natürlich von der Art der Verabreichung und den Behandlungsbedingungen ab.

Die Erfindung sei an folgenden Beispielen erläutert :

Beispiel 1

Aclacinomycin wurde, gelöst in Wasser, in einer Dosis von 0,1 mg/kg bis 1,0 mg/kg Körpergewicht Auszuchtmäusen (Stamm NMRI) intravenös appliziert. Gleichzei- tig wurden als Antigen $10^6$ oder $10^9$ Schaferythrozyten intravenös verabreicht. 5 Tage später wurden 2 x $10^8$ Schaferythrozyten subcutan in das Bindegewebe eines Füßchens injiziert, und 24 Stunden später wurden die Mäuse entblutet, das Serum gewonnen und die Antikörpertiter gegen Schaferythrozyten durch die dem Fachmann bekannten Hämagglutinations- und Hämolysemethoden bestimmt.

Die Ergebnisse sind in der Tabelle I zusammengefaßt:

T a b e l l e   I

Immunsuppressive Wirkung von Aclacinomycin (ACM)

| appliziertes ACM (mg/kg) i.v. | Schaferythrozyten i.v. injiziert zur Immunisierung | Hämagglutination Endtiter ($-\lg_2$) | | Hämolyse Endtiter ($-\lg_2$) | |
|---|---|---|---|---|---|
| | | $10^6$ | $10^9$ | $10^6$ | $10^9$ |
| 0,1 | | 0*) | 7,5 | 0 | 9 |
| 0,5 | | 0 | 7,4 | 3,5 | 9,5 |
| 1,0 | | 0 | 8,3 | 5,5 | 9,3 |
| Kontrolle (keine Applikation) *) 10 Tiere pro Gruppe | | 7,5 | 8 | 6,5 | 8 |

Wie aus dieser Tabelle ersichtlich, verringert Aclacinomycin speziell in niedrigen Dosierungen die Antikörperbildung gegen Schaferythrozyten, wirkt also immunsuppressiv.

Beispiel 2

Wirkung von Aclacinomycin auf die Anzahl Antikörper-(Plaque-)bildender Zellen (PFC).

NMRI-Mäuse wurden entsprechend Beispiel 1 mit $10^6$ Schaferythrozyten (SRBC) und ACM immunisiert und nach 5 Tagen mit $2 \times 10^8$ SRBC restimuliert. 12 Tage nach der ersten Antigengabe wurde die Milz entnommen und die Anzahl der Plaque-bildenden Zellen pro Milz bestimmt. Mit der dem Fachmann bekannten Methode nach Jerne wurden sowohl die direkten als auch die indirekten PFC bestimmt und daraus der Anteil der anti-SRBC-IgM bzw. -IgG produzie-renden Zellen bestimmt.

- 5 -

Die Ergebnisse sind in der Tabelle II zusammengefaßt:


T a b e l l e  II


Anzahl der Plaque-forming cells / Milz
(Tag 12 nach Immunisierung)

| mg ACM/kg Körpergewicht | IgM-PFC | IgG-PFC |
|---|---|---|
| 0,5 | 3931 | 60 900 |
| 0,1 | 2063 | 8 800 |
| 0,01 | 1068 | 7 600 |
| Kontrolle | 5644 | 32 300 |


Wie aus der Tabelle II ersichtlich ist, reduziert ACM
in niedrigen Dosen sowohl die IgM- als auch die IgG-PFC
und ist somit als immunsuppressiv zu bezeichnen.

Patentansprüche :

1. Ein Immunsuppressivum bestehend aus Aclacinomycin und einem Applikationshilfsmittel.

2. Ein Immunsuppressivum nach Anspruch 1 enthaltend pro Dosis 5 mg bis 0,0001 µg Aclacinomycin pro kg Körpergewicht.

0058387

Nummer der Anmeldung

**Europäisches Patentamt**

# EUROPÄISCHER RECHERCHENBERICHT

EP 82 10 0990

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | THE JOURNAL OF ANTIBIOTICS, Band XXVIII, Nr. 10, 1975, Seiten 830-834, Edit. Jap. Antibiot. Res. Assoc. TOSHIKAZU OKI et al.: "New anti-tumor antibiotics, aclacinomycins A and B" * Insgesamt * | 1 | C 07 H 15/24<br>A 61 K 31/70 |

-----

| | | | RECHERCHIERTE SACHGEBIETE (Int. Cl. 3) |
|---|---|---|---|
| | | | C 07 H 15/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 10-05-1982 | RAJIC M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein-stimmendes Dokument

EPA Form 1503. 03 82